# EUROPEAN PATENT APPLICATION

(11) **EP 4 520 388 A1**
(43) Date of publication of application: **12.03.2025**
(21) Application number: 23818924.5
(22) Date of filing: 19.05.2023
(51) Int. Cl.: A61M 60/81

(54) **DRIVING APPARATUS AND BLOOD PUMP**

(30) Priority: 10.06.2022 CN 202210650440
(71) Applicant: Shenzhen Core Medical Technology Co., Ltd., Shenzhen, Guangdong 518000 (CN)
(72) Inventor: XIE, Duanqing, Shenzhen, Guangdong 518000 (CN); YU, Shunzhou, Shenzhen, Guangdong 518000 (CN)
(74) Representative: Appleyard Lees IP LLP
(86) International application number: PCT/CN2023/095268
(87) International publication number: WO 2023/236759

(57) **Abstract**

A blood pump (1) and a driving apparatus (20). The driving apparatus (20) comprises a housing assembly (100) and a rotating shaft (200). The housing assembly (100) is provided with a mounting hole (131). The rotating shaft (200) comprises a first shaft segment (210), and an arc-shaped convex surface (211) is formed circumferentially on the first shaft segment (210). At least a portion of the arc-shaped convex surface (211) is positioned within the mounting hole (131). An inflection point of the arc-shaped convex surface (211) is opposite to the wall of the mounting hole (131). At the inflection point, the gap between the arc-shaped convex surface (211) and the wall of the mounting hole (131) is minimized. When the first shaft segment (210) is in contact with the wall of the mounting hole (131), the inflection point is in contact with the wall of the mounting hole (131).

## Description

### CROSS-REFERENCE TO RELATED APPLICATIONS

This application claims priority to Chinese Patent Application No. CN 202210650440.6, filed on June 10, 2022, the entire content of which is incorporated herein by reference in its entirety.

### TECHNICAL FIELD

The present application relates to the technical field of medical devices, and in particular to a driving device and a blood pump.

### BACKGROUND

An intravascular blood pump is a blood pumping device that can be inserted into a patient's heart through the patient's blood vessels. The intravascular blood pump is placed in an opening of a heart valve, so that blood can flow through the blood pump and into an arterial blood vessel. The blood pump includes a driving device and an impeller. The impeller is fixed to a rotating shaft of the driving device, and the impeller is driven to rotate through the rotating shaft. However, for the conventional driving device, the rotating shaft usually has great wear.

### SUMMARY

Accordingly, the present application provides a driving device and a blood pump, which can reduce wear of a rotating shaft.

According to a first aspect, the present application provides a driving device configured to drive an impeller to rotate, the driving device includes:
a housing assembly provided with a mounting hole; and
a rotating shaft including a first shaft section that is fixedly connected to the impeller and rotatably extends through the mounting hole, wherein the first shaft section is provided with an arc-shaped convex surface on a circumference thereof, the arc-shaped convex surface is partially located in the mounting hole, an inflection point of the arc-shaped convex surface is opposite to a hole wall of the mounting hole, and a gap between the arc-shaped convex surface and the hole wall of the mounting hole is minimum at the inflection point of the arc-shaped convex surface, when the first shaft section is in contact with the hole wall of the mounting hole, the inflection point of the arc-shaped convex surface is in contact with the hole wall of the mounting hole.

According to a second aspect, the present application provides a blood pump including an impeller and a driving device, the driving device includes:
a housing assembly provided with a mounting hole; and
a rotating shaft including a first shaft section that is fixedly connected to the impeller and rotatably extends through the mounting hole, wherein the first shaft section is provided with an arc-shaped convex surface on a circumference thereof, the arc-shaped convex surface is partially located in the mounting hole, an inflection point of the arc-shaped convex surface is opposite to a hole wall of the mounting hole, and a gap between the arc-shaped convex surface and the hole wall of the mounting hole is minimum at the inflection point of the arc-shaped convex surface, when the first shaft section is in contact with the hole wall of the mounting hole, the inflection point of the arc-shaped convex surface is in contact with the hole wall of the mounting hole;
wherein the impeller is fixedly connected to the first shaft section, and the impeller is capable of rotating along with the rotating shaft.

Details of one or more embodiments of the present application are set forth in the following drawings and descriptions. Other features, objects and advantages of the present application will become apparent with reference to the specification, drawings, and claims.

### BRIEF DESCRIPTION OF THE DRAWINGS

In order to illustrate the embodiments of the present application more clearly, the drawings used in the embodiments will be described briefly. Apparently, the following described drawings are merely for the embodiments of the present application, and other drawings can be derived by those of ordinary skill in the art without any creative effort.
FIG. 1 is a perspective view of a blood pump according to a first embodiment.
FIG. 2 is an exploded view of the blood pump shown in FIG. 1 with a part of a cannula assembly omitted.
FIG. 3 is a cross-sectional view of the blood pump shown in FIG. 1 with a part of a cannula assembly omitted.
FIG. 4 is a perspective view of a first shaft sleeve of the blood pump shown in FIG. 1.
FIG. 5 is a perspective view of a second shaft sleeve of the blood pump shown in FIG. 1.
FIG. 6 is a cross-sectional view illustrating the first shaft sleeve and the second shaft sleeve of the blood pump shown in FIG. 1 assembled.
FIG. 7 is a perspective view of a shaft tube of the blood pump shown in FIG. 1.
FIG. 8 is a cross-sectional view illustrating the first shaft sleeve, the second shaft sleeve and the rotating shaft of the blood pump shown in FIG. 1 assembled.
FIG. 9 is a schematic view of a portion A in FIG. 8.
FIG. 10 is a schematic view of a rotor of the blood pump shown in FIG. 1.
FIG. 11 is a cross-sectional view of the rotor shown in FIG. 10.
FIG. 12 is an exploded view of the rotor shown in FIG. 10.
FIG. 13 is a partial cross-sectional view of a driving device of a blood pump according to a second embodiment.
FIG. 14 is a partial cross-sectional view of a driving device of a blood pump according to a third embodiment.
FIG. 15 is a partial cross-sectional view of a driving device of a blood pump according to a fourth embodiment.
FIG. 16 is a partial cross-sectional view of a driving device of a blood pump according to a fifth embodiment.

### DETAILED DESCRIPTION OF THE EMBODIMENTS

In order to make the objectives, technical solutions, and advantages of the present application more clearly understood, the present application is described in further detail below in conjunction with the accompanying drawings and embodiments. It should be understood that specific embodiments described herein are intended only to interpret the present application and not intended to limit the present application.

It should be noted that when one element is referred to as "fixed to" or "arranged on" another element, it may be directly disposed on the other element or an intermediate element may exist. When one element is considered to be "connected to" another element, it may be directly connected to the other element or indirectly connected to the another element.

In addition, the terms "first" and "second" are used for descriptive purposes only, which cannot be construed as indicating or implying a relative importance, or implicitly specifying the number of the indicated technical features. Thus, the features defined with "first" and "second" may explicitly or implicitly include one or more features. In the description of the present application, "a plurality of" means two or more, unless specifically stated otherwise.

In order to illustrate the technical solutions of the present application, description will be made below with reference to specific drawings and embodiments.

Herein, "a proximal end" is defined as an end adjacent to an operator; and "a distal end" is defined as an end away from the operator.

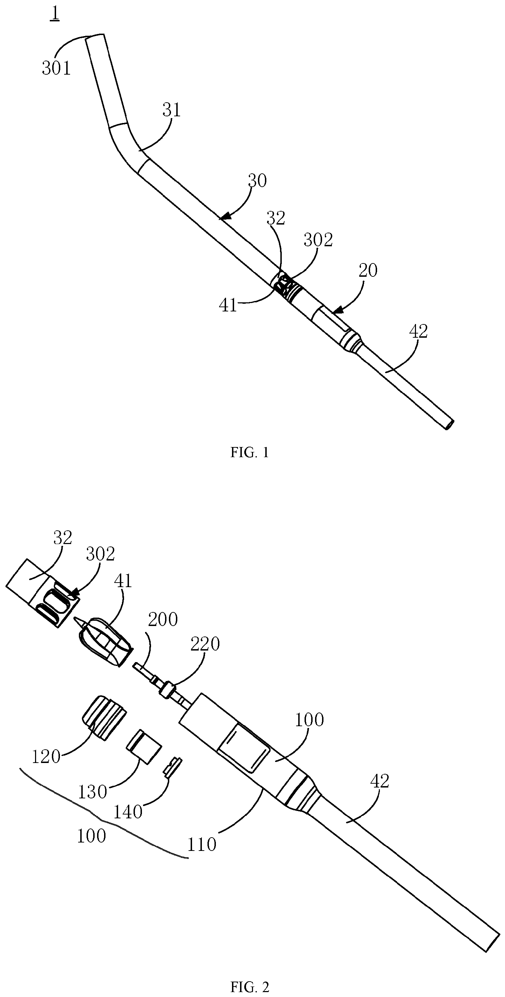

Referring to FIGS. 1 and 2, a blood pump 1 according to an embodiment of the present application includes a driving device 20, a cannula assembly 30, an impeller 41, and a catheter 42. The cannula assembly 30 is connected to a distal end of the driving device 20. The catheter 42 is connected to a proximal end of the driving device 20. The impeller 41 is rotatably provided in the cannula assembly 30. The impeller 41 is connected to the driving device 20. The driving device 20 can drive the impeller 41 to rotate, so as to realize a blood pumping function of the blood pump 1.

Specifically, the cannula assembly 30 is provided with an inlet 301 and an outlet 302. The outlet 302 is closer to the driving device 20 than the inlet 301. That is, the outlet 302 is located at a proximal end of the cannula assembly 30, and the inlet 301 is located at a distal end of the cannula assembly 30. In an embodiment, the cannula assembly 30 extends through a heart valve, such as an aortic valve, while the inlet 301 is located within a heart, and the outlet 302 and the driving device 20 are located in a blood vessel outside the heart, such as an aorta. When the impeller 41 rotates, blood flows into the cannula assembly 30 from the inlet 301, and then flows out of the cannula assembly 30 from the outlet 302 to enter the blood vessel such as the aorta.

In some embodiments, the cannula assembly 30 includes an inserting tube 31 and an outlet tube 32 that are fixedly connected to each other. The outlet tube 32 is connected between the inserting tube 31 and the driving device 20, i.e., a distal end of the outlet tube 32 is connected to a proximal end of the inserting tube 31, and a proximal end of the outlet tube 32 is connected to the driving device 20. The inlet 301 is formed in the inserting tube 31, and the outlet 302 is formed in the outlet tube 32. The impeller 41 is received in the outlet tube 32. A position of the impeller 41 substantially corresponds to a position of the outlet 302.

The catheter 42 is butted against an end of driving device 20 away from cannula assembly 30. The catheter 42 is configured to accommodate various supply pipelines, and the supply pipelines may be, for example, cleaning pipelines configured to introduce cleaning liquid into the driving device 20, or wires configured to supply power to the driving device 20, or a support component configured to support the catheter 42.

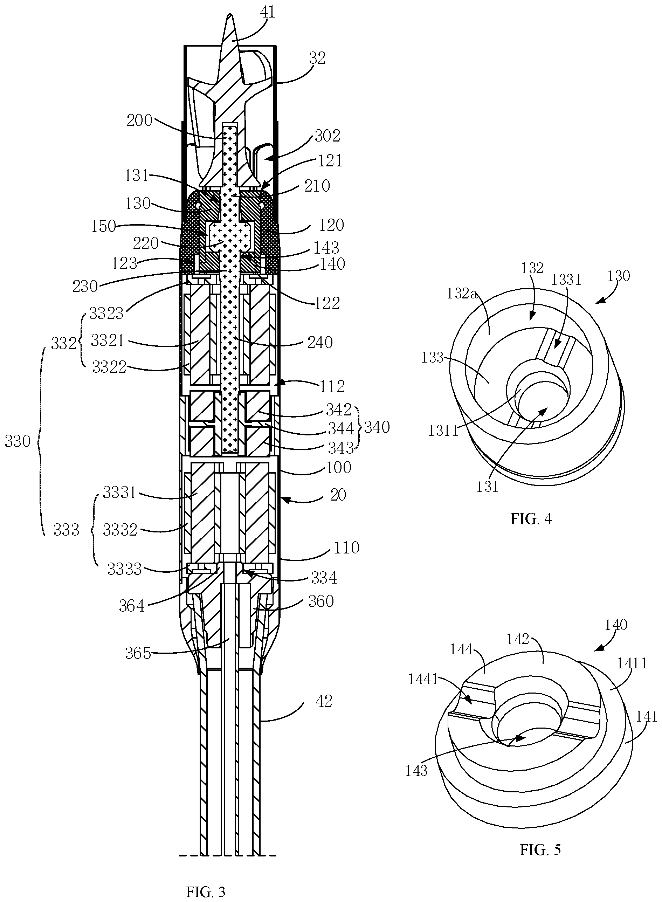

Referring to FIG. 3, in some embodiments, the driving device 20 includes a housing assembly 100, a rotating shaft 200, a stator 330, and a rotor 340. The housing assembly 100 is provided with a mounting hole 131, and the rotating shaft 200 rotatably extends through the mounting hole 131. The impeller 41 is fixedly connected to the rotating shaft 200. Both the stator 330 and the rotor 340 are received in the housing assembly 100. The rotor 340 is fixedly connected to the rotating shaft 200. The stator 330 can drive the rotor 340 to rotate, the rotor 340 can drive the rotating shaft 200 to rotate, and the impeller 41 can rotate along with the rotating shaft 200 to realize the blood pumping function of the blood pump 1. The wires in the catheter 42 extend into the housing assembly 100 and are electrically connected to stator 330 to provide power to the stator 330.

In the illustrated embodiment, the housing assembly 100 includes a pump housing 110, a shaft tube 120, a first shaft sleeve 130, and a second shaft sleeve 140. The pump housing 110, the shaft tube 120, the first shaft sleeve 130, and the second shaft sleeve 140 are separated prior to assembly, that is, the housing assembly 100 is formed by assembling the pump housing 110, the shaft tube 120, the first shaft sleeve 130, and the second shaft sleeve 140 that are separated.

The pump housing 110 is a cylindrical structure having a substantially circular cross section. The pump housing 110 is provided with a receiving cavity 112, and both the stator 330 and the rotor 340 are received in the receiving cavity 112 of the pump housing 110.

One end of the shaft tube 120 is fixedly connected to the pump housing 110, and the other end of the haft tube 120 is fixedly connected to the cannula assembly 30 (specifically, the outlet tube 32). The shaft tube 120 has a mounting opening 121 that is located at an end of the shaft tube 120 adjacent to the cannula assembly 30.

The first shaft sleeve 130 and the second shaft sleeve 140 are fixedly received in the shaft tube 120. Both the first shaft sleeve 130 and the second shaft sleeve 140 are fixedly connected to the shaft tube 120. The first shaft sleeve 130 and the second shaft sleeve 140 are arranged along an axial direction of the shaft tube 120, and the first shaft sleeve 130 is closer to the impeller 41 than the second shaft sleeve 140. The mounting hole 131 is formed on the first shaft sleeve 130. The rotating shaft 200 rotatably extends through the first shaft sleeve 130 and the second shaft sleeve 140. Specifically, the first shaft sleeve 130 and the second shaft sleeve 140 are separated. Both the first shaft sleeve 130 and the second shaft sleeve 140 can be mounted from the mounting opening 121 of the shaft tube 120, that is, the maximum outer diameters of both the first shaft sleeve 130 and the second shaft sleeve 140 are slightly less than a diameter of the mounting opening 121.

Specifically, an end of the shaft tube 120 adjacent to the pump housing 110 is provided with a bearing protrusion 122, and the second shaft sleeve 140 abuts against the bearing protrusion 122. The first shaft sleeve 130 abuts against the second shaft sleeve 140, so that the bearing protrusion 122 limit the first shaft sleeve 130 and the second shaft sleeve 140 in an axial direction of the pump housing 110, so as to facilitate the assembly of the first shaft sleeve 130 and the second shaft sleeve 140. In an embodiment, the bearing protrusion 122 is substantially annular. The shaft tube 120 is further provided with a glue injection hole 123, and the glue injection hole 123 is filled with adhesive. The adhesive fixedly bonds the first shaft sleeve 130 and the second shaft sleeve 140 to the shaft tube 120, and fixes the first shaft sleeve 130 and the second shaft sleeve 140.

Referring to FIG. 3, FIG. 4, FIG. 5 and FIG. 6, the first shaft sleeve 130 is provided with a limiting hole 132, and a diameter of the limiting hole 132 is greater than that of the mounting hole 131. The mounting hole 131 is closer to the impeller 41 than limiting hole 132. The limiting hole 132 and the mounting hole 131 are coaxially arranged and are in communication with each other, so that the limiting hole 132 and the mounting hole 131 cooperatively form a stepped hole. The first shaft sleeve 130 has a first limiting surface 133 that defines a partial boundary of the limiting hole 132. The first limiting surface 133 may be perpendicular to an axial direction of the rotating shaft 200, or may be inclined relative to the axial direction of the rotating shaft 200.

The second shaft sleeve 140 includes a thick section 141 and a thin section 142. A size of a cross section of the thin section 142 is less than a size of a cross section of the thick section 141. The thick section 141 has an abutting surface 1411. In the illustrated embodiment, outer contours of both the thin section 142 and the thick section 141 are both circular. In this case, a size of a cross section of the thin section 142 being less than a size of a cross section of the thick section 141 means that an outer diameter of the thin section 142 is less than an outer diameter of the thick section 141. The thin section 142 protrudes from the abutting surface 1411. The second shaft sleeve 140 is provided with a through hole 143 extending from an end surface of an end of the thin section 142 away from the abutting surface 1411 to a side of the thick section 141 away from the abutting surface 1411, so that the through hole 143 extends through the thick section 141 and the thin section 142. A diameter of the limiting hole 132 is greater than that of the through hole 143. The thin section 142 is received in the limiting hole 132, and the abutting surface 1411 abuts against the first shaft sleeve 130. The mounting accuracy and mounting efficiency can be improved by the limiting action of the abutting surface 1411 and the guiding action of the thin section 142. The side of the thick section 141 away from the abutting surface 1411 abuts against the bearing protrusion 122. The end surface of the end of the thin section 142 away from the abutting surface 1411 is a second limiting surface 144, and the second limiting surface 144 is spaced apart from and opposite to the first limiting surface 133, so that the first limiting surface 133 and the second limiting surface 144 are spaced along the axial direction of the rotating shaft 200. The second limiting surface 144 may be perpendicular to the axial direction of the rotating shaft 200, or may be inclined relative to the axial direction of the rotating shaft 200. In the illustrated embodiment, both the first limiting surface 133 and the second limiting surface 144 are perpendicular to the axial direction of the rotating shaft 200, and the first limiting surface 133 and the second limiting surface 144 are parallel and opposite to each other.

A hole wall 132a of the limiting hole 132, the first limiting surface 133, and the second limiting surface 144 cooperatively define a limiting cavity 150. The mounting hole 131 and the through hole 143 are both in communication with the limiting cavity 150. In other words, the limiting cavity 150 is actually a part of the limiting hole 132, and the first limiting surface 133 and the second limiting surface 144 are two cavity walls of the limiting cavity 150 in the axial direction of the rotating shaft 200.

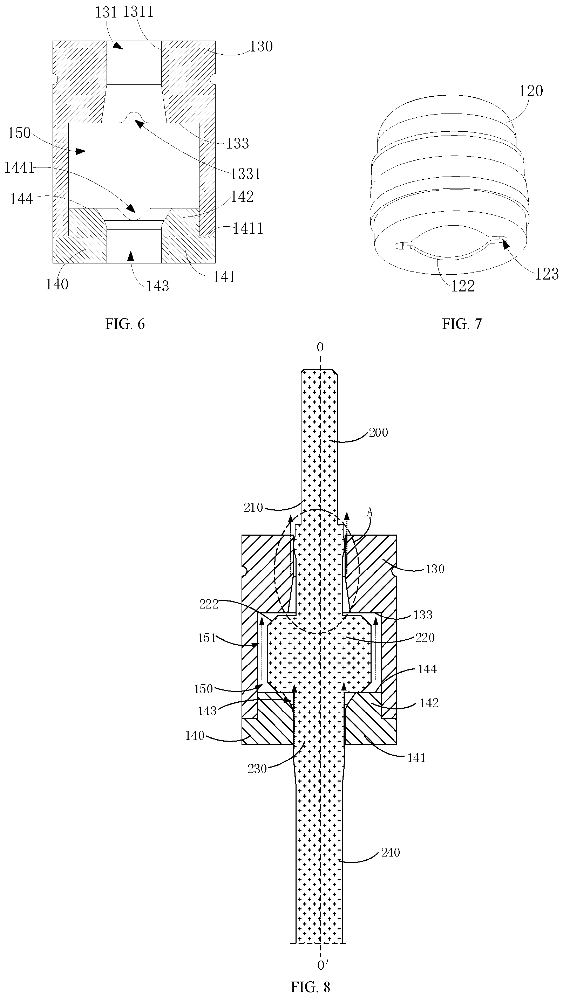

The rotating shaft 200 extends through the mounting hole 131, the limiting cavity 150, and the through hole 143. One end of the rotating shaft 200 is received in the pump housing 110, and the other end of the rotating shaft 200 extends into the cannula assembly 30 and is fixedly connected to the impeller 41. Gaps are provided between the rotating shaft 200 and a hole wall of the mounting hole 131, between the rotating shaft 200 and a cavity wall of the limiting cavity 150, and between the rotating shaft 200 and a hole wall of the through hole 143 for the cleaning liquid to flow. The cleaning liquid can flow from the pump housing 110 through the gap between the rotating shaft 200 and the hole wall of the through hole 143, the gap between the rotating shaft 200 and the cavity wall of the limiting cavity 150, and the gap between the rotating shaft 200 and the mounting hole 131 to enter the cannula assembly 30, and then flow out of the outlet 302. Dashed arrows in FIG. 8 represents a flow direction of the cleaning liquid, which is opposite to a flow direction of the blood in the cannula assembly 30, so that the blood in the cannula assembly 30 can be prevented from entering the driving device 20 through the mounting hole 131. In addition, the cleaning liquid further servers a lubrication, and the cleaning liquid can reduce the frictional resistance between the rotating shaft 200 and the first shaft sleeve 130 and the frictional resistance between the rotating shaft 200 and the second shaft sleeve 140, and reduce the wear between the rotating shaft 200 and the first shaft sleeve 130 and the wear between the rotating shaft 200 and the second shaft sleeve 140.

It should be noted that the housing assembly 100 is not limited to the above manner. In some embodiments, two or more of the pump housing 110, the shaft tube 120, the first shaft sleeve 130, and the second shaft sleeve 140 may be integrally formed. For example, the pump housing 110 and the shaft tube 120 may be integrally formed. For example, one of the first shaft sleeve 130 and the second shaft sleeve 140 may be integrally formed with the shaft tube 120. For example, the first shaft sleeve 130 may be a disc-shaped ring structure. For example, the first shaft sleeve 130 is composed of a tubular ring and disc-shaped ring that are separated. For example, the second shaft sleeve 140 may also have only the thick section 141.

Referring to figs. 3, 7, 8, and 9, in some embodiments, the rotating shaft 200 includes a first shaft section 210, a second shaft section 220, a third shaft section 230, and a fourth shaft section 240 that are sequentially connected. Axes of the first shaft section 210, the second shaft section 220, the third shaft section 230, and the fourth shaft section 240 coincide with each other. The first shaft section 210 rotatably extends through the mounting hole 131, and the first shaft section 210 is fixedly connected to the impeller 41. The second shaft section 220 is rotatably received in the limiting cavity 150. The third shaft section 230 rotatably extends through the through hole 143. The fourth shaft section 240 is received in the receiving cavity 112 of the pump housing 110. The fourth shaft section 240 is fixedly connected to the rotor 340.

A portion of the first shaft section 210 is received in the housing assembly 100, and a portion of the first shaft section 210 extends into the cannula assembly 30 and is fixedly connected to the impeller 41. The first shaft section 210 is provided with an arc-shaped convex surface 211 in a circumference thereof. Specifically, the arc-shaped convex surface 211 protrudes radially in a direction away from an axis of the first shaft section 210 (a radial direction is a direction perpendicular to the axis of the first shaft section 210). The arc-shaped convex surface 211 is partially located in the mounting hole 131, and an inflection point of the arc-shaped convex surface 211 is opposite to the hole wall of the mounting hole 131, and the gap between the arc-shaped convex surface 211 and the hole wall of the mounting hole 131 is minimum at the inflection point of the arc-shaped convex surface 211. When the first shaft section 210 is in contact with the hole wall of the mounting hole 131, the inflection point of the arc-shaped convex surface 211 is in contact with the hole wall of the mounting hole 131. Since the rotating shaft 200 swings radially to a certain extent during rotation, when the rotating shaft 200 swings, the first shaft section 210 will be in contact with the hole wall of the mounting hole 131. A contact area between the first shaft section 210 and the hole wall of the mounting hole 131 is larger, the wear of the first shaft section 210 will be greater. According to the above-mentioned rotating shaft 200, the arc-shaped convex surface 211 is provided on the circumference of the first shaft section 210, so that when the first shaft section 210 is in contact with the hole wall of the mounting hole 131, only the inflection point of the arc-shaped convex surface 211 is in contact with the hole wall of the mounting hole 131, so as to form a point-surface contact, thereby reducing the contact area between the first shaft section 210 and the hole wall of the mounting hole 131 and reducing the wear of the rotating shaft 200.

It should be noted that the inflection point of the arc-shaped convex surface 211 of the present application refers to a point where the arc-shaped convex surface 211 has the maximum distance from an axis OO' of the first shaft section 210, that is, the most convex point of the arc-shaped convex surface 211. For example, in the illustrated embodiment, the inflection point of the arc-shaped convex surface 211 is at line PP'. Since the axes of the first shaft section 210, the second shaft section 220, the third shaft section 230, and the fourth shaft section 240 coincide with each other, the axis OO' of the first shaft section 210 is the axis of the rotating shaft 200, and is also the axis of the second shaft section 220, the axis of the third shaft section 230, and the axis of the fourth shaft section 240.

Specifically, the arc-shaped convex surface 211 is arranged for one circle around the axis OO' of the first shaft section 210, and the arc-shaped convex surface 211 in this manner can facilitate the manufacturing of the rotating shaft 200. In the illustrated embodiment, the arc-shaped convex surface 211 is continuously arranged for one circle around the axis of the first shaft section 210. It should be understood that, in other embodiments, a plurality of arc-shaped convex surfaces 211 spaced apart may also be provided in the axial direction of the first shaft section 210.

In some embodiments, a width of a gap between the inflection point of the arc-shaped convex surface 211 (line PP') and the hole wall of the mounting hole 131 is less than or equal to 2 µm. Since the minimized red blood cells (approximately 8 µm in diameter and approximately 2 µm in thickness) are difficult to enter the gap with a width of less than or equal to 2 µm, and the cleaning liquid of the backwash passes through the gap, the blood is prevented from entering the gap.

Specifically, along the axial direction of the first shaft section 210, a distance between the inflection point (line PP') of the arc-shaped convex surface 211 and a plane where an opening of an end of the mounting hole 131 adjacent to the impeller 41 is located is defined as H, and H is H ≤ 0.2 mm. In the illustrated embodiment, the plane where the opening of the end of the mounting hole 131 adjacent to the end of the impeller 41 is perpendicular to the axial direction of the first shaft section 210, in other words, the plane where the opening of the end of the mounting hole 131 adjacent to the end of the impeller 41 is perpendicular to the axis OO' of the first shaft section 210. Further, 0.1 mm ≤ H ≤ 0.2 mm, the inflection point (line PP') of the arc-shaped convex surface 211 is located in the mounting hole 131 and is slightly lower than the opening of the end of the mounting hole 131 adjacent to the impeller 41. On the one hand, the hole wall of the mounting hole 131 forms a better supporting effect on the rotating shaft 200, so that when the rotating shaft 200 is in contact with the hole wall of the mounting hole 131, only the inflection point (line PP') of the arc-shaped convex surface 211 is in contact with the hole wall of the mounting hole 131, and on the other hand, the flushing force of the cleaning liquid is ensured.

Specifically, the arc-shaped convex surface 211 has a first arc surface portion 212 and a second arc surface portion 213 connected to the first arc surface portion 212. The first arc surface portion 212 and the second arc surface portion 213 are arranged along the axial direction of the first shaft section 210, and a connection portion between the first arc surface portion 212 and the second arc surface portion 213 is the inflection point (line PP') of the arc-shaped convex surface 211. Along the axial direction of the first shaft section 210 approaching the impeller 41, a distance from the first arc surface portion 212 to the axis OO' of the first shaft section 210 gradually increases, and a distance between the second arc surface portion 213 and the axis OO' of the first shaft section 210 gradually decreases. In the illustrated embodiment, the arc-shaped convex surface 211 is completely located in the mounting hole 131. Along the axis OO' of the first shaft section 210 approaching the impeller 41, a width of the gap between the first arc surface portion 212 and the hole wall of the mounting hole 131 gradually decreases, a width of the gap between the second arc surface portion 213 and the hole wall of the mounting hole 131 gradually increases, and at a connection portion of the first arc surface portion 212 and the second arc surface portion 213, that is, at the line PP', a width of the gap between the arc-shaped convex surface 211 and the hole wall of the mounting hole 131 is minimized.

In some embodiments, in order to prevent red blood cells in the blood from entering between the first shaft section 210 and the hole wall of the mounting hole 131, a width of the gap between a hole wall of the opening of the end of the mounting hole 131 adjacent to the impeller 41 and the first shaft section 210 is less than or equal to 2 µm. In this case, the width of the gap between the inflection point (line PP') of the arc-shaped convex surface 211 and the hole wall of the mounting hole 131 is less than 2 µm, that is, the width of the gap between the inflection point (line PP') of the arc-shaped convex surface 211 and the hole wall of the mounting hole 131 is less than the width of the gap between the hole wall of the opening of the end of the mounting hole 131 adjacent to the impeller 41 and the first shaft section 210.

It should be understood that the width of the gap between the inflection point of the arc-shaped convex surface 211 and the hole wall of the mounting hole 131, and the width of the gap between the hole wall of the opening of the end of the mounting hole 131 adjacent to the impeller 41 and the first shaft section 210 may also be adjusted as required and designed.

In order to facilitate the cleaning liquid to enter the mounting hole 131 from the limiting cavity 150, the mounting hole 131 has a first hole portion 131a and a second hole portion 131b that are in communication with each other. A diameter of the first hole portion 131a is constant. A diameter of the second hole portion 131b gradually decreases along a direction approaching the first hole portion 131a. The first shaft section 210 extends through the first hole portion 131a and the second hole portion 131b. The inflection point (line PP') of the arc-shaped convex surface 211 is opposite to a hole wall of the first hole portion 131a. When the first shaft section 210 is in contact with the hole wall of the mounting hole 131, the inflection point (line PP') of the arc-shaped convex surface 211 is in contact with the hole wall of the first hole portion 131a. That is, a diameter of an end of the second hole portion 131b adjacent to the limiting cavity 150 is greater than the diameter of the first hole portion 131a. The first hole portion 131a having a constant diameter can better support the rotating shaft 200, and reduce a swing radian of the rotating shaft 200. The second hole portion 131b having a variable diameter in the above manner can guide the cleaning liquid, so as to facilitate the cleaning liquid to enter the mounting hole 131.

In some embodiments, an inner chamfer is formed on a hole wall of an end of the through hole 143 adjacent to the limiting cavity 150, which helps to reduce the contact area between the rotating shaft 200 and the second shaft sleeve 140 and reduce the wear of the rotating shaft 200.

The second shaft section 220 is fixedly connected to an end of the first shaft section 210 away from the impeller 41. The second shaft section 220 is rotatably received in the limiting cavity 150. In other words, a size of the cross section of the second shaft section 220 is less than a size of the cross section of the limiting cavity 150. A diameter of the cross section of the second shaft section 220 is greater than the diameter of the mounting hole 131 and the diameter of the through hole 143, so that the second shaft section 220 is limited in the limiting cavity 150. In this way, the second shaft section 220 does not enter the through hole 143 and the mounting hole 131, and the second shaft section 220 is located between the first limiting surface 133 and the second limiting surface 144, thereby limiting the rotating shaft 200 in the axial direction of the rotating shaft 200.

Specifically, the second shaft section 220 is capable of abutting against the first limiting surface 133 and the second limiting surface 144 to prevent the rotating shaft 200 from moving axially or limit an axial movement distance of the rotating shaft 200. In some embodiments, the second shaft section 220 is always in sliding contact with the first limiting surface 133 and the second limiting surface 144. In other embodiments, a distance between the first limiting surface 133 and the second limiting surface 144 is slightly greater than an axial length of the second shaft section 220, so that during rotation of the rotating shaft 200, the second shaft section 220 has a certain floating space between the first limiting surface 133 and the second limiting surface 144 for the cleaning liquid to flow. A flow gap 151 for the cleaning liquid to flow is formed between the second shaft section 220 and the cavity wall of the limiting cavity 150 extending along the axis OO'.

Referring to FIG. 4, FIG. 5 and FIG. 6 again, further, the first limiting surface 133 is partially recessed to form a first flow guiding groove 1331. The first flow guiding groove 1331 extends from the hole wall 1311 of the mounting hole 131 to the hole wall 132a of the limiting hole 132, so that the first flow guiding groove 1331 is in communication with the mounting hole 131 and the limiting hole 132. Since the limiting cavity 150 is a part of the limiting hole 132, the first flow guiding groove 1331 is also in communication with the limiting cavity 150. The second limiting surface 144 is partially recessed to form a second flow guiding groove 1441. he second flow guiding groove 1441 extends from the hole wall 1311 of the through hole 143 to an outer peripheral surface of the thin section 142 and is in communication with the through hole 143 and the limiting cavity 150. Since the flow gap 151 is actually a part of the limiting cavity 150, the flow gap 151 is in communication with the first flow guiding groove 1331 and the second flow guiding groove 1441 at the same time. The first flow guiding groove 1331 and the second flow guiding groove 1441 facilitate the circulation of the cleaning liquid.

It should be noted that, in some embodiments, only one of the first flow guiding groove 1331 and the second flow guiding groove 1441 may be provided, or the first flow guiding groove 1331 and the second flow guiding groove 1441 may not be provided.

In some embodiments, chamfers 222 are formed on both end portions of the second shaft section 220 in the axial direction thereof. In this way, on one hand, a contact area between the rotating shaft 200 and the first limiting surface 133 and/or the second limiting surface 144 is reduced, and the contact area between the rotating shaft 200 and the first shaft sleeve 130 and the second shaft sleeve 140 is further reduced, so as to further reduce the wear of the rotating shaft 200. On the other hand, sharp edges of the second shaft section 220 in contact with the first shaft sleeve 130 and the second shaft sleeve 140 are prevented from forming wear on the first shaft sleeve 130 and the second shaft sleeve 140, and the flow guiding effect on the cleaning liquid can also be formed.

The third shaft section 230 rotatably extends through the through hole 143. A gap is provided between the third shaft section 230 and the hole wall of the through hole 143 for the cleaning liquid to flow.

The fourth shaft section 240 is connected to an end of the third shaft section 230 away from the second shaft section 220, and the fourth shaft section 240 is received in the receiving cavity 112. A size of a cross section of the fourth shaft section 240 is less than a size of a cross section of the third shaft section 230. The rotor 340 is fixedly connected to the fourth shaft section 240. The fourth shaft section 240 is at least partially received in the stator 330.

Specifically, the rotating shaft 200, the first shaft sleeve 130, and the second shaft sleeve 140 may be made of a ceramic material, so that wear resistance of the rotating shaft 200, the first shaft sleeve 130, and the second shaft sleeve 140 can be improved, and the rotating shaft 200, the first shaft sleeve 130, and the second shaft sleeve 140 may be further prevented from being worn.

Referring to FIG. 3 again, the stator 330 includes a first stator unit 332 and a second stator unit 333. Both the first stator unit 332 and the second stator unit 333 can drive the rotor 340 to rotate. Specifically, the first stator unit 332 and the second stator unit 333 are spaced apart along an extending direction of the rotating shaft 200. Both the first stator unit 332 and the second stator unit 333 are fixedly connected to the housing assembly 100. The fourth shaft section 240 of the rotating shaft 200 rotatably extends through the first stator unit 332. That is, the rotor 340 is rotatable relative to the housing assembly 100, while the first stator unit 332 and the second stator unit 333 are not rotatable relative to the housing assembly 100.

The first stator unit 332 and the second stator unit 333 may be connected in parallel or in series. In some embodiments, the first stator unit 332 and the second stator unit 333 are capable of synchronously driving the rotor 340 to rotate. The first stator unit 332 and the second stator unit 333 can cooperatively drive the rotor 340 to rotate, or can separately drive the rotor 340 to rotate.

In some embodiments, the rotor 340 is magnetic and the stator 330 is capable of generating a rotating magnetic field configured to drive the rotor 340 to rotate. Specifically, both the first stator unit 332 and the second stator unit 333 can generate rotating magnetic fields configured to drive the rotor 340 to rotate.

Specifically, the first stator unit 332 includes a first magnetic core 3321, a first coil 3322, and a first back plate 3323. The first back plate 3323 is fixedly connected to the housing assembly 100. In the illustrated embodiment, the first back plate 3323 is fixedly connected to the shaft tube 120. A plurality of first magnetic cores 3321 are provided, and the plurality of first magnetic cores 3321 are spaced apart along a circumference. Specifically, an extending direction of each first magnetic core 3321 is consistent with an extending direction of the rotating shaft 200. Each first magnetic core 3321 is fixedly connected to the first back plate 3323. The first coil 3322 is wound around the first magnetic core 3321. One first coil 3322 and one first magnetic core 3321 form a coil winding. In this case, a plurality of coil windings of the first stator unit 332 are provided around the fourth shaft section 240.

A structure of the second stator unit 333 is similar to that of the first stator unit 332. The second stator unit 333 includes a second magnetic core 3331, a second coil 3332, and a second back plate 3333. The second back plate 3333 is fixedly connected to the housing assembly 100. A plurality of second magnetic cores 3331 are provided, and the plurality of second magnetic cores 3331 are spaced apart along a circumference. Specifically, each the second magnetic cores 3331 extends in a direction parallel to the axis (i.e., the axis OO') of the fourth shaft section 240. Each second magnetic core 3331 is fixedly connected to the second back plate 3333. The second coil 3332 is wound around the second magnetic core 3331. One second coil 3332 and one second magnetic core 3331 form a coil winding. In this case, a plurality of coil windings of the second stator unit 333 are provided around the axis (i.e., OO') of the fourth shaft section 240.

In some embodiments, the first magnetic core 3321 and the second magnetic core 3331 each include a magnetic column and a head portion (i.e., a pole shoe) disposed at an end of the magnetic column. An extending direction of the magnetic column is consistent with the extending direction of the rotating shaft. The first back plate 3323 is engaged with an end of the magnetic column of the first magnetic core 3321 away from the head. The second back plate 3333 is engaged with an end of the magnetic column of the second magnetic core 3331 away from the head. In the extending direction of the magnetic column, the magnetic column is substantially a columnar body of uniform size, that is, a size of a cross-section of the magnetic column 3331 remains constant. In general, the magnetic column 3331 is uniform in thickness. The first coil 3322 is wound around the magnetic column of the first magnetic core 3321, and the second coil 3332 is wound around the magnetic column of the second magnetic core 3331.

Referring to FIG. 3, in the illustrated embodiment, the first magnetic core 3321 and the second magnetic core 3331 each include only the magnetic column, that is, neither the first magnetic core 3321 nor the second magnetic core 3331 has the head portion (i.e., the pole shoe) with a large width in cross-section. In this case, the magnetic column of the first stator unit 332 is the first magnetic core 3321, and the magnetic column of the second stator unit 333 is the second magnetic core 3331. At this time, the entire first magnetic core 3321 can be magnetically coupled with the rotor 340, and the entire second magnetic core 3331 can be magnetically coupled with the rotor 340. Compared with the magnetic core having the pole shoe, the magnetic core having only the magnetic column can reduce the magnetic loss, and increase the magnetic coupling density between the magnetic core and the rotor 340 to increase the torque of the stator unit to the rotor 340 under the same current. On the other hand, the magnetic core without the head portion can also greatly reduce the power reduction of the driving device 20 due to the local magnetic short circuit caused by the contact between adjacent magnetic cores.

It should be understood that the first magnetic core 3321 and the second magnetic core 3331 are not limited to the above two manners. In some embodiments, one of the first magnetic core 3321 and the second magnetic core 3331 may have both the magnetic column and the head portion, and the other one has only the magnetic column.

In some embodiments, shapes of the cross sections of the magnetic columns of the first magnetic core 3321 and the second magnetic core 3331 are substantially triangular prism-shaped, and an edge of each magnetic column faces the axis of the rotating shaft. In some embodiments, the edges of the magnetic columns are rounded, that is, the edges of the magnetic columns are relatively smooth and blunt rounded edges, so as to eliminate the sharp edges on the magnetic columns, which not only facilitates the subsequent winding of the coil, but also facilitates the protection of the insulating material coated on the coil. In other embodiments, the shapes of the cross sections of the magnetic columns of the first magnetic core 3321 and the second magnetic core 3331 may also be a sector, a circle, a trapezoid, a sector ring, etc.

In the illustrated embodiment, along the axis of the fourth shaft section 240 (i.e., along the axis OO'), the rotating shaft 200 is spaced apart from the second stator unit 333, that is, the end of the fourth shaft section 240 away from the third shaft section 230 is spaced apart from the second stator unit 333, that is, the fourth shaft section 240 of the rotating shaft 200 does not extend into the second stator unit 333. A size of a cross section of the magnetic column of the second stator unit 333 is greater than a size of a cross section of the magnetic column of the first stator unit 332.

An area of the cross section of the magnetic column is larger, the generated magnetic flux is larger, the torque of the stator unit to the rotor 340 is larger, and the required current the smaller, which is conducive to reducing the power consumption and reducing the heat generation. In the case that an area of the cross section of the first stator unit 332 is the same as an area of the cross section of the second stator unit 333 and an outer diameter of the housing assembly 100 remains constant, since the rotating shaft 200 is located outside the second stator unit 333, and the rotating shaft 200 does not extend into the second stator unit 333, the size of the cross section of the magnetic column of the second stator unit 333 can be reasonably increased in a manner without increasing an outer diameter of the pump housing 110. In this way, a driving torque of the second stator unit 333 to the rotor 340 can be increased. When the required torque is the same, a current supply to the stator 330 can be reasonably reduced in such a manner, so that a power consumption is reduced, and the heat generated by the driving device 20 is also reduced, so as to avoid discomfort or even injury to the human body due to excessively high temperature caused by the heat accumulation during the operation of the blood pump 1.

It should be noted that, in other embodiments, the rotating shaft 200 may also extend into the second stator unit 333. At this time, the sizes of the cross sections of the magnetic columns of the first stator unit 332 and the second stator unit 333 are the same.

The first back plate 3323 and the second back plate 3333 are substantially plate-shaped. The first back plate 3323 and the second back plate 3333 are made of the same material as the first magnetic core 3321 and the second magnetic core 3331, for example, a soft magnetic material such as cobalt steel.

The back plate can serve to close a magnetic circuit of the stator unit, so as to promote and increase generation of the magnetic flux of the stator unit and improve a coupling capability between each stator unit and the rotor 340. In other words, the first back plate 3323 provided in the first stator unit 332 can promote and increase the generation of the magnetic flux of the first stator unit 332, and improve the coupling capacity between the first stator unit 332 and the rotor 340. The second back plate 3333 provided in the second stator unit 333 can promote and increase generation of the magnetic flux of the second stator unit 333, and improve a coupling capability between the second stator unit 333 and the rotor 340. Since the back plate can increase the magnetic flux, providing back plates in the first stator unit 332 and the second stator unit 333 respectively also facilitates reducing the overall diameter of the driving device 20.

Specifically, the driving device 20 further includes a positioning member 360 fixedly connected to the pump housing 110. The positioning member 360 includes a positioning column 364. The second back plate 3333 of the second stator unit 333 is provided with a positioning hole 3334, and the positioning column 364 extends through the positioning hole 3334. In this way, the positioning member 360 can perform the function of positioning the second stator unit 333, thereby improving the mounting accuracy and the mounting efficiency of the second stator unit 333. Specifically, a central axis of the positioning column 364 and a central axis of the second stator unit 333 coincide with each other. In some embodiments, the positioning member 360 is further provided with a through hole 365, and the through hole 365 may be in communication with the cleaning pipeline for introducing cleaning liquid into the driving device 20 or for mounting the cleaning pipeline.

It should be noted that, in some embodiments, the first stator unit 332 may not have the first back plate 3323, and the second stator unit 333 may not have the second back plate 3333. Alternatively, one of the first stator unit 332 and the second stator unit 333 has a back plate, and the other one has no back plate. If the second stator unit 333 does not have the second back plate 3333, a plurality of positioning holes may be directly formed on the positioning member 360, and one ends of the plurality of second magnetic cores 3331 are positioned in the plurality of positioning holes, respectively.

In some embodiments, the positioning member 360 may be omitted. In this case, a clamping position configured to be engaged with an edge of the second back plate 3333 may be provided in the pump housing 110, so as to fix the second stator unit 333 through the clamping position engaged with the second back plate 3333. Alternatively, the second stator unit 333 may be adhered to the pump housing 110 by an adhesive. The first stator unit 332 may be fixed to the shaft tube 120 by an adhesive, or may be fixed to the first back plate 3323 by providing a corresponding clamping position in the pump housing 110.

Referring to figs. 3 and 10 to 12, the rotor 340 is received in the receiving cavity 112 of the pump housing 110. In the illustrated embodiment, the rotor 340 is located between the first stator unit 333 and the second stator unit 334 along the axis OO'. Specifically, the rotor 340 includes a first magnet 342 and a second magnet 343 that are both fixedly connected to the fourth shaft section 240. Both the first magnet 342 and the second magnet 343 are located between the first stator unit 332 and the second stator unit 333, that is, the first stator unit 332, the first magnet 342, the second magnet 343, and the second stator unit 333 are sequentially arranged along the axis OO'. The first stator unit 332 can generate a rotating magnetic field configured to drive the first magnet 342 to rotate, and the second stator unit 333 can generate a rotating magnetic field configured to drive the second magnet 343 to rotate. The two stator units provide torque to the rotor 340 through two magnets, respectively, which can increase the driving force for rotating the rotor 340.

Specifically, the rotor 340 further includes a flywheel 344 fixedly connected to the fourth shaft section 240 of the rotating shaft 200. The flywheel 344 is located between the first stator unit 332 and the second stator unit 333, and both the first magnet 342 and the second magnet 343 are fixedly connected to the flywheel 344. More specifically, the flywheel 344 is fixedly connected to an end of the fourth shaft section 240 away from the third shaft section 230.

By arranging the flywheel 344, a connection strength between the first magnet 342 and the fourth shaft section 240 and a connection strength between the second magnet 343 and the fourth shaft section 240 can be enhanced. In addition, by arranging both the first magnet 342 and the second magnet 343 on the same flywheel 344, the shaking of the fourth shaft section 240 during rotation can be reduced, and the fourth shaft section 240 is more stable during rotation.

In the illustrated embodiment, the flywheel 344 includes an inner tube 3442, a disc-shaped portion 3444, and an outer ring wall 3446. Both the inner tube 3442 and the outer ring wall 3446 are of circular tubular structures, and the disc-shaped portion 3444 is of an annular disc structure. Both the inner tube 3442 and the outer ring wall 3446 are fixedly connected to the disc-shaped portion 3444. The outer ring wall 3446 surrounds the disc-shaped portion 3444, the inner tube 3442 and the outer ring wall 3446 are arranged coaxially, and the fourth shaft section 240 extends through the inner tube 3442 and is fixedly connected to the inner tube 3442. A receiving space is formed between the inner tube 3442 and the outer ring wall 3446, and the disc-shaped portion 3444 divides the receiving space into two mounting cavities 3448. Both mounting cavities 3448 are annular cavities. The first magnet 342 and the second magnet 343 are received in the two mounting cavities 3448, respectively. Both the first magnet 342 and the second magnet 343 are annular, and the shapes of the two mounting cavities 3448 are respectively adapted to the first magnet 342 and the second magnet 343, so as to facilitate the mounting and positioning of the first magnet 342 and the second magnet 343. Such arrangement enables the flywheel 344 to limit the first magnet 342 and the second magnet 343, which not only facilitates the mounting of the first magnet 342 and the second magnet 343, but also enables the first magnet 342 and the second magnet 343 to be combined with the flywheel 344 more stably.

It should be noted that the flywheel 344 is not limited to the above structure. In some embodiments, the flywheel 344 does not have the outer ring wall 3446. In some embodiments, the flywheel 344 does not have the outer ring wall 3446 and the inner tube 3442. In this case, the fourth shaft section 240 fixedly extends through the disc-shaped portion 3444, e.g., a center of the disc-shaped portion 3444. Compared with the flywheel 344 having only the disc-shaped portion 3444, the provision of the inner tube 3442 enables the flywheel 344 to be more stably connected to the fourth shaft section 240.

In some embodiments, the first magnet 342 and the second magnet 343 are both annular Halbach array magnets. Specifically, the first magnet 342 and the second magnet 343 each include a plurality of magnetic bodies, for example, four, six, eight or ten, etc. magnetic bodies. Each magnetic body is in a shape of a sector ring. The plurality of magnetic bodies of the first magnet are arranged for one circle around the fourth shaft section 240 to form a ring structure. The plurality of magnetic bodies of the second magnet 343 are arranged for one circle around the rotor 340 to form a ring structure.

More specifically, the first magnet 342 has a first magnetic body 3422 magnetized along an axial direction of the first magnet 342, the second magnet 343 has a second magnetic body 3432 magnetized along the axial direction of the second magnet 343, the first magnetic body 3422 and the second magnetic body 3432 are provided on opposite sides of the disc-shaped portion 3444, respectively, and positions of the first magnetic body 3422 and the second magnetic body 3432 correspond to each other. In an extending direction of the rotor 340, polarities of the first magnetic body 3422 and the second magnetic body 3432 on a side facing the disc-shaped portion 3444 are opposite. Such arrangement can facilitate the mounting of the first magnet 342 and the second magnet 343, and avoid the problem that the first magnetic body 3422 and the second magnetic body 3432 corresponding to the disc-shaped portion 3444 of the first magnet 342 and the second magnet 343 repel each other and cause difficulty in assembly. For example, when the polarity of the first magnetic body 3422 on the side facing the disc-shaped portion 3444 is N pole, the polarity of the second magnetic body 3432 on the side facing the disc-shaped portion 3444 is S pole. According to the principle that the N pole and the S pole attract each other, the interference of the magnetic repulsion force is eliminated, and the mounting efficiency of the first magnet 342 and the second magnet 343 is improved.

In order to facilitate the mounting of the first magnet 342 and the second magnet 343 and improve the mounting accuracy of the first magnet 342 and the second magnet 343, the flywheel 344 is further provided with a mark portion 345 configured to determine a mounting position of the first magnetic body 3422 and a mounting position of the second magnetic body 4332. The mark portion 345 may be provided as a groove, a scale line, or a mark, etc. When the first magnet 342 and the second magnet 343 are mounted, as long as the position of one of the first magnet 342 and the second magnet 343 is marked by the mark portion 445, the mounting position of the remaining magnetic body can be determined, thereby facilitating the mounting of the first magnet 342 and the second magnet 343. Specifically, the mark portion 345 is provided on at least one of the inner tube 3442, the disc-shaped portion 3444, and the outer ring wall 3446. Specifically, in the illustrated embodiment, end surfaces of both ends of the inner tube 3442 are provided with the mark portions 345.

The above-mentioned driving device and blood pump have at least the following advantages:
(1) Since the rotating shaft 200 swings radially to a certain extent during rotation, when the rotating shaft 200 swings, the rotating shaft 200 will be in contact with the hole wall of the mounting hole 131. A contact area between the rotating shaft 200 and the hole wall of the mounting hole 131 is larger, the wear of the rotating shaft 200 will be greater, the arc-shaped convex surface 211 is provided on the circumference of the first shaft section 210 of the rotating shaft 200 of the driving device 20, and the gap between the arc-shaped convex surface 211 and the hole wall of the mounting hole 131 is minimized at the inflection point PP' of the arc-shaped convex surface 211, so that when the first shaft section 210 is in contact with the hole wall of the mounting hole 131 of the housing assembly 100, the inflection point PP' of the arc-shaped convex surface 211 is in contact with the hole wall of the mounting hole 131, so as to form a point-surface contact, thereby reducing the contact area between the first shaft section 210 and the wall of the mounting hole 131 and reducing the wear of the rotating shaft 200.
(2) The split-type housing assembly 100 with the above-mentioned structure is adopted, i.e., the pump housing 110, the shaft tube 120, the first shaft sleeve 130, and the second shaft sleeve 140 are assembled into the housing assembly 100, and the maximum outer diameter of the mounting opening 121 of the shaft tube 120 is slightly less than the diameter of the mounting hole 121, which can facilitate the assembly of the driving device 20. For example, the first shaft sleeve 130, the second shaft sleeve 140, and the rotating shaft 200 can be assembled from one direction, the assembly of the drive device can be simplified, and the production efficiency can be improved.
(3) Since the fourth shaft section 240 of the rotating shaft 200 extends through the stator 330, and the size of the cross section of the fourth shaft section 240 is less than the size of the cross section of the third shaft section 230, on the basis of ensuring the structural strength of the entire rotating shaft 200, a space occupied by the fourth shaft section 240 in the radial direction of the stator 330 can be reduced. Under the condition that the outer diameters of the stator 330 and the pump housing 110 remain unchanged, the size of the cross section of the magnetic column in the stator 330 can be reasonably increased. In general, the magnetic column can be reasonably thickened. The area of the cross section of the magnetic column is larger, the generated magnetic flux is larger, the torque of the stator 330 to the rotor 340 is larger, and the required current the smaller, which is conducive to reducing the power consumption and reducing the heat generation, and avoiding discomfort or even injury to the human body due to excessively high temperature caused by the heat accumulation during the operation of the blood pump 1. By making the size of the cross section of the third shaft section 230 large, the rotating shaft 200 can have a large structural strength at the through hole 143.
(4) While the rotating shaft 200 of the driving device 20 is spaced apart from the second stator unit 333 in the axial direction, the size of the cross section of the magnetic column of the second stator unit 333 can be reasonably increased in a manner without increasing the outer diameter of the pump housing 110. In this way, the driving torque of the second stator unit 333 to the rotor 340 can be increased. When the required torque is the same, the current supply to the stator 330 can be reduced in such a manner, so that the power consumption is reduced, and the heat generated by the driving device 20 is reduced.

It should be understood that the structure of the driving device 20 is not limited thereto. In some embodiments, the fourth shaft section 240 of the rotating shaft 200 also extends through the second stator unit 333. In another embodiment, the stator 330 may have only one stator unit, may have only the first stator unit 332, or may have only the second stator unit 333.

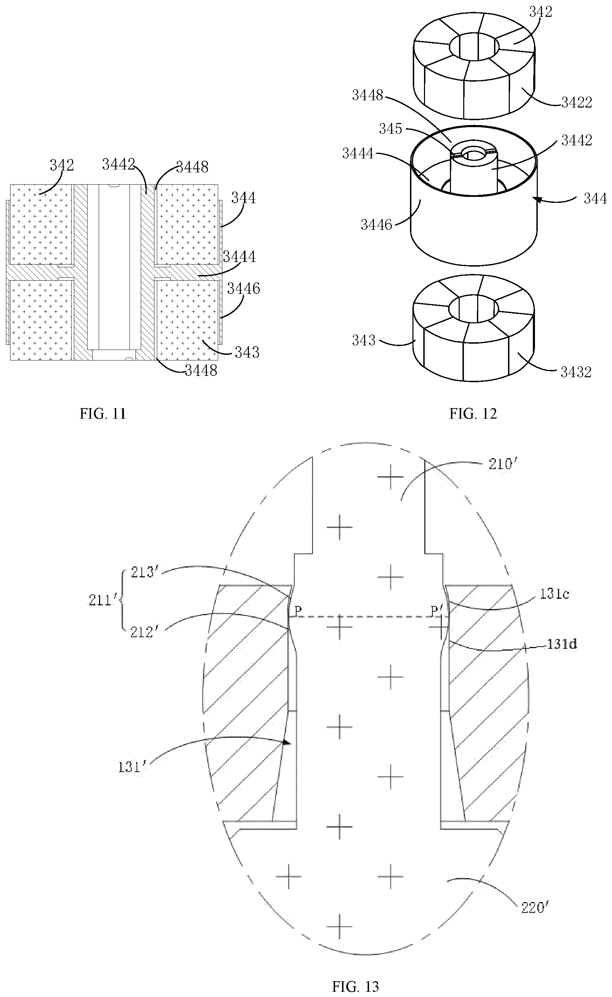

Referring to FIG. 13, a structure of a driving device of a blood pump according to a second embodiment is substantially the same as that of the driving device 20 according to the first embodiment, and differences mainly lie in:
A hole wall of a mounting hole 131' of this embodiment is provided with an arc-shaped concave surface portion 131c opposite to an arc-shaped convex surface 211', a curvature of the concave surface portion 131c is less than a curvature of the arc-shaped convex surface 211'. When the first shaft section 210' is in contact with the hole wall of the mounting hole 131', the inflection point (line PP') of the arc-shaped convex surface 211' is still in contact with the concave surface portion 131c to form a point-surface contact.

In the illustrated embodiment, a position of the concave surface portion 131c is opposite to a position of a second arc surface portion 213' of the arc-shaped convex surface 211'. The curvature of the concave surface portion 131c is less than a curvature of the second arc surface portion 213'. The hole wall of the mounting hole 131' further includes a first inner wall 131d opposite to a first arc surface portion 212'. The first inner wall 131d is a straight wall extending parallel to an axis of a first shaft section 210'. The first inner wall 131d may also be an inclined wall inclined relative to the axis of the first shaft section 210', or the first inner wall 131d is an arc-shaped concave wall. When the hole wall of the mounting hole 131' opposite to the first arc surface portion 212', i.e., a first inner wall 131d, is an arc-shaped concave wall, a curvature of the hole wall of the mounting hole 131' opposite to the first arc surface portion 212' may be the same as or different from the curvature of the concave portion 131c. The first arc surface portion 212' is closer to a second shaft section 220' of the rotating shaft than the second arc surface portion 213'.

Since the driving device of this embodiment has a structure similar to that of the driving device of the first embodiment, the driving device of this embodiment and the blood pump having the driving device of the second embodiment also have effects similar to those of the first embodiment.

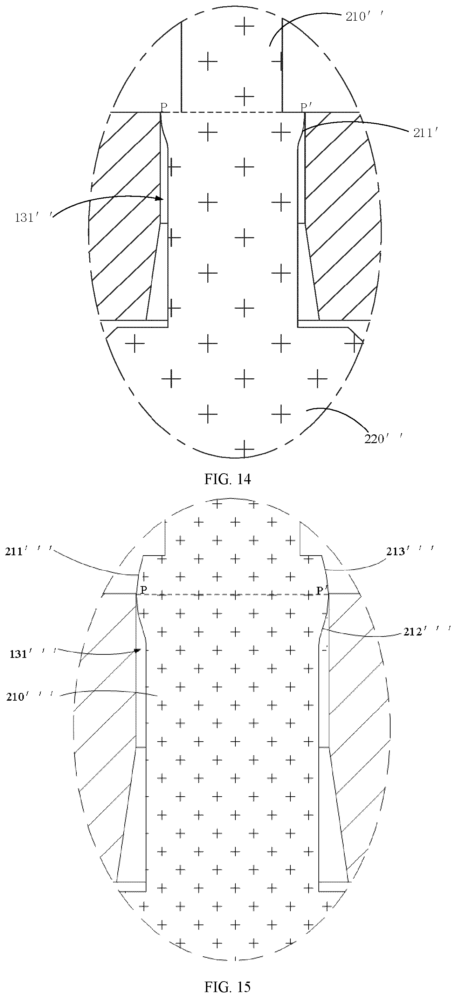

Referring to FIG. 14, a structure of a driving device of a blood pump according to a third embodiment is substantially the same as that of the driving device 20 according to the first embodiment, and differences mainly lie in:
In this embodiment, along an axial direction of a first shaft section 210" approaching the impeller, a width of a gap between an arc-shaped convex surface 211" and the hole wall of the mounting hole 131" gradually decreases. That is, compared with the arc-shaped convex surface 211 on the first shaft section 210 of the driving device 20 of the first embodiment, the arc-shaped convex surface 211" of this embodiment has only the first arc-shaped surface portion. At this time, a position of an inflection point PP" of the arc-shaped convex surface 211" is located at an end of the arc-shaped convex surface 211" away from a second shaft section 220". In the illustrated embodiment, the position of the inflection point PP" of the arc-shaped convex surface 211" is exactly coplanar with a plane where an opening of an end of a mounting hole 131" adjacent to the impeller is located.

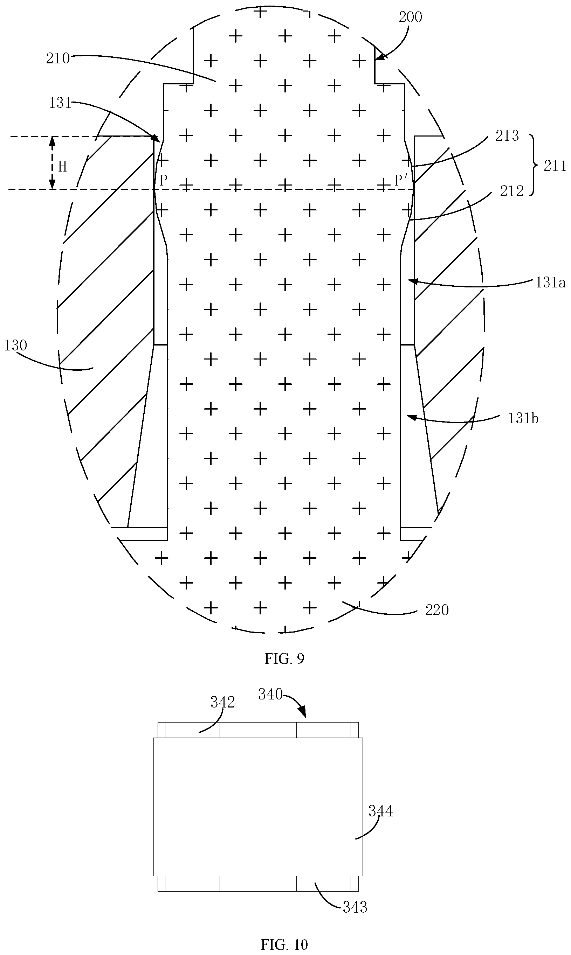

It should be noted that, in some embodiments, the position of the inflection point PP' of the arc-shaped convex surface 211" may be lower than the plane where an opening of an end of a mounting hole 131" adjacent to the impeller is located, the position of the inflection point PP' of the arc-shaped convex surface 211" is still received in the mounting hole 131", and the opening of the end of the mounting hole 131" adjacent to the impeller is closer to the impeller than the position of the inflection point PP" of the arc-shaped convex surface 211". That is, similar to FIG. 9, a distance H between the position of the inflection point PP' of the arc-shaped convex surface 211" and the plane where the opening of an end of the mounting hole 131" adjacent to the impeller satisfies 0.1 mm ≤ H ≤0.2 mm.

Since the driving device of this embodiment has a structure similar to that of the driving device of the first embodiment, the driving device of this embodiment and the blood pump having the driving device of the second embodiment also have effects similar to those of the first embodiment.

Referring to FIG. 15, a structure of a driving device of a blood pump according to a fourth embodiment is substantially the same as that of the driving device 20 according to the first embodiment, and differences mainly lie in:
In this embodiment, an arc-shaped convex surface 211‴ on a first shaft section 210‴ has a structure similar to that of the arc-shaped convex surface 211 of the first embodiment, and also has a first arc surface portion 212‴ and a second arc surface portion 213‴ connected to the first arc surface portion 212‴. The first arc surface 212‴ of the arc convex surface 211‴ is located in a mounting hole 131‴, and the second arc surface 213‴ is partially located outside the mounting hole 131"'. A distance H (similar to that shown in FIG. 9) between the position of the inflection point PP' of the arc-shaped convex surface 211‴ and a plane where an opening of an end of the mounting hole 131‴ adjacent to the impeller is less than or equal to 0.2 mm, and further 0.1 mm ≤ H ≤ 0.2 mm.

Since the driving device of this embodiment has a structure similar to that of the driving device of the first embodiment, the driving device of this embodiment and the blood pump having the driving device of this embodiment also have effects similar to those of the first embodiment.

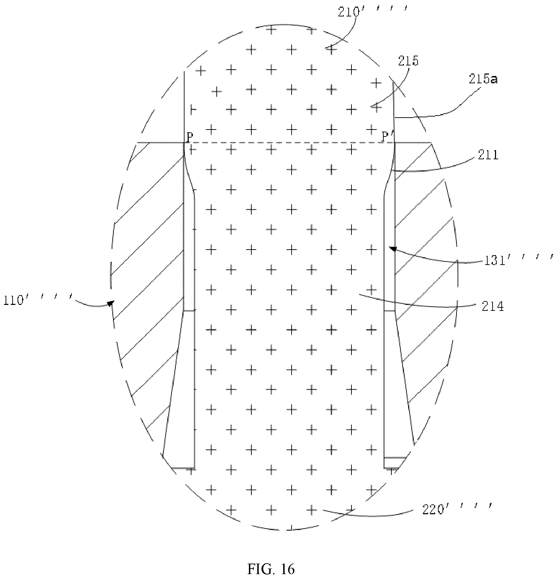

Referring to FIG. 16, a structure of the driving device of the blood pump according to a fifth embodiment is substantially the same as that of the driving device 20 according to the first embodiment, and differences mainly lie in:
In the illustrated embodiment, a first shaft section 210ʺʺ includes a first column portion 214 received in a housing assembly 110ʺʺ and a second column portion 215 connected to the first column portion 214 and located outside the housing assembly 110. The second column portion 215 is configured to fixedly connect to the impeller, and the second column portion 215 and the first column portion 214 are coaxially arranged. A circumferential surface of an end of the second column portion 215 adjacent to the first column portion 214 is a cylindrical surface 215a. An arc-shaped convex surface 211ʺʺ is located at an end of the first column portion 214 adjacent to the second column portion 215. The arc-shaped convex surface 211ʺʺ is connected to the cylindrical surface 215a. A connection portion between the arc-shaped convex surface 211ʺʺ and the cylindrical surface 215a is an inflection point PP' of the arc-shaped convex surface 211ʺʺ. The inflection point PP' of the arc-shaped convex surface 211ʺʺ is coplanar with a plane where an opening of an end of the mounting hole 131ʺʺ adjacent to the impeller. Specifically, a diameter of the end of the second column portion 215 adjacent to the first column portion 214 is equal to a diameter of the first column portion 214 at the inflection point PP' of the arc-shaped convex surface 211"". That is, in this embodiment, the arc-shaped convex surface 211"" still has only the first arc surface portion. The arc-shaped convex surface 211ʺʺ is located at an end of the first column portion 214 away from the second shaft section 220"".

Since the driving device of this embodiment has a structure similar to that of the driving device of the first embodiment, the driving device of this embodiment and the blood pump having the driving device of this embodiment also have effects similar to those of the first embodiment.

The foregoing embodiments are merely intended to describe the technical solutions of the present disclosure, but not to limit the present disclosure. Although the present disclosure is described in detail with reference to the foregoing embodiments, those of ordinary skill in the art should understand that they may still make modifications to the technical solutions described in the foregoing embodiments or make equivalent replacements to some technical features thereof, without departing from the spirit and scope of the technical solutions of the embodiments of the present disclosure, all of which fall within the protection scope of the present disclosure.

## Claims

1. A driving device configured to drive an impeller to rotate, the driving device comprising:
a housing assembly provided with a mounting hole; and
a rotating shaft comprising a first shaft section that is fixedly connected to the impeller and rotatably extends through the mounting hole, wherein the first shaft section is provided with an arc-shaped convex surface on a circumference thereof, the arc-shaped convex surface is partially located in the mounting hole, an inflection point of the arc-shaped convex surface is opposite to a hole wall of the mounting hole, and a gap between the arc-shaped convex surface and the hole wall of the mounting hole is minimum at the inflection point of the arc-shaped convex surface, when the first shaft section is in contact with the hole wall of the mounting hole, the inflection point of the arc-shaped convex surface is in contact with the hole wall of the mounting hole.

2. The driving device according to claim 1, wherein along an axial direction of the first shaft section, a distance between the inflection point of the arc-shaped convex surface and a plane where an opening of an end of the mounting hole adjacent to the impeller is located is defined as H, and H ≤ 0.2 mm.

3. The driving device according to claim 1, wherein the arc-shaped convex surface has a first arc surface portion located in the mounting hole, along an axial direction of the first shaft section approaching the impeller, a distance between the first arc surface portion and an axis of the first shaft section gradually increases, and the inflection point of the arc-shaped convex surface is located on the first arc surface portion.

4. The driving device according to claim 3, wherein the arc-shaped convex surface further has a second arc surface portion connected to the first arc surface portion, the second arc surface portion and the first arc surface portion are arranged along the axial direction of the first shaft section, a connection portion between the second arc surface portion and the first arc surface portion is the inflection point of the arc-shaped convex surface, and a distance between the second arc surface portion and the axis of the first shaft section gradually decreases along the axial direction of the first shaft section approaching the impeller.

5. The driving device according to claim 4, wherein the second arc surface portion is located in the mounting hole.

6. The driving device according to claim 1, wherein a position of the inflection point of the arc-shaped convex surface is coplanar with a plane where an opening of an end of a mounting hole adjacent to the impeller is located.

7. The driving device according to claim 6, wherein the first shaft section comprises a first column portion received in the housing assembly and a second column portion connected to the first column portion and located outside the housing assembly, the second column portion is configured to fixedly connect to the impeller, the second column portion and the first column portion are coaxially arranged, a circumferential surface of an end of the second column portion adjacent to the first column portion is a cylindrical surface, the arc-shaped convex surface is located at an end of the first column portion adjacent to the second column portion, the arc-shaped convex surface is connected to the cylindrical surface, and a connection portion between the arc-shaped convex surface and the cylindrical surface is the inflection point of the arc-shaped convex surface.

8. The driving device according to claim 1, wherein the arc-shaped convex surface is completely located in the mounting hole, and a width of the gap between the arc-shaped convex surface and the hole wall of the mounting hole along an axial direction of the rotating shaft approaching the impeller gradually decreases.

9. The driving device according to claim 1, wherein the arc-shaped convex surface is continuously arranged for one circle around an axis of the first shaft section.

10. The driving device according to claim 1, wherein a width of the gap between the inflection point of the arc-shaped convex surface and the hole wall of the mounting hole is less than or equal to 2 µm.

11. The driving device according to claim 1, wherein the hole wall of the mounting hole is provided with an arc-shaped concave surface portion opposite to the arc-shaped convex surface, a curvature of the concave surface portion is less than a curvature of the arc-shaped convex surface, when the first shaft section is in contact with the hole wall of the mounting hole, the inflection point of the arc-shaped convex surface is in contact with the concave surface portion.

12. The driving device according to claim 11, wherein the arc-shaped convex surface has a first arc surface portion and a second arc surface portion, the first arc surface portion and the second arc surface portion are arranged along an axial direction of the first shaft section, the second arc surface portion is closer to the impeller than the first arc surface portion, and a connection portion between the second arc surface portion and the first arc surface portion is the inflection point of the arc-shaped convex surface;
a position of the concave surface portion is opposite to a position of the second arc surface portion, and the curvature of the concave surface portion is less than a curvature of the second arc surface portion.

13. The driving device according to claim 12, wherein the hole wall of the mounting hole comprises a first inner wall opposite to the first arc surface portion;
wherein the first inner wall is a straight wall parallel to an axis of the first shaft section, or the first inner wall is an inclined wall inclined relative to the axis of the first shaft section, or the first inner wall is an arc-shaped concave wall.

14. The driving device according to claim 1, wherein the mounting hole has a first hole portion and a second hole portion that are in communication with each other, a diameter of the first hole portion is constant, a diameter of the second hole portion gradually decreases along a direction approaching the first hole portion, the first shaft section extends through the first hole portion and the second hole portion, the inflection point of the arc-shaped convex surface is opposite to a hole wall of the first hole portion, when the first shaft section is in contact with the hole wall of the mounting hole, the inflection point of the arc-shaped convex surface is in contact with the hole wall of the first hole portion.

15. The driving device according to claim 1, wherein the rotating shaft further comprises a second shaft section connected to an end of the first shaft section, and a third shaft section connected to the second shaft section away from the first shaft section, and an end of the first shaft section away from the second shaft section is configured to be fixedly connected to the impeller;
the housing assembly is further provided with a limiting cavity and a through hole, the limiting cavity is in communication with the mounting hole, the through hole is in communication with the limiting cavity, the second shaft section is rotatably received in the limiting cavity, the third shaft section rotatably extends through the through hole, and a size of a cross section of the second shaft section is greater than a diameter of the mounting hole and a diameter of the through hole.

16. The driving device according to claim 15, wherein the housing assembly is further provided with a receiving cavity in communication with the through hole, the rotating shaft further has a fourth shaft section connected to an end of the third shaft section away from the second shaft section, the fourth shaft section is received in the receiving cavity, the fourth shaft section is thinner than the third shaft section, the driving device further comprises a rotor and a stator that are received in the receiving cavity, the rotor is fixedly connected to the fourth shaft section, the stator is capable of driving the rotor to rotate, the rotor is capable of driving the rotating shaft to rotate, and the fourth shaft section is at least partially received in the stator.

17. The driving device according to claim 16, wherein the rotor is magnetic, the stator comprises a first stator unit and a second stator unit, both the first stator unit and the second stator unit are capable of generating rotating magnetic fields configured to drive the rotor to rotate, the first stator unit, the rotor, and the second stator unit are sequentially arranged along an axis of the fourth shaft section, the fourth shaft section rotatably extends through the first stator unit, the rotor is fixedly connected to an end of the fourth shaft section away from the third shaft section, and the second stator unit and the rotating shaft are spaced apart along the axis of the fourth shaft section;
wherein the first stator unit and the second stator unit each have a plurality of coil windings, the plurality of coil windings of the first stator unit are provided around the fourth shaft section, the plurality of coil windings of the second stator unit are provided around the axis of the fourth shaft section, the plurality of coil windings of the second stator unit and the plurality of coil windings of the first stator unit each have a magnetic column, and a size of the cross section of the magnetic column of the second stator unit is greater than a size of the cross section of the magnetic column of the first stator unit.

18. The driving device according to claim 16, wherein the housing assembly comprises a pump housing, a shaft tube, a first shaft sleeve, and a second shaft sleeve that are separated, the pump housing is provided with the receiving cavity, an end of the shaft tube is fixedly connected to the pump housing, an end of the shaft tube adjacent to the pump housing is provided with a bearing protrusion, and an end of the shaft tube away from the pump housing is provided with a mounting opening;
the first shaft sleeve and the second shaft sleeve are capable of fixedly receiving in the shaft tube from the mounting opening and are arranged along an axial direction of the shaft tube, the first shaft sleeve is closer to the impeller than the second shaft sleeve, and the second shaft sleeve abuts against the bearing protrusion;
the mounting hole is provided on the first shaft sleeve, and the rotating shaft rotatably extends through the first shaft sleeve and the second shaft sleeve.

19. The driving device according to claim 15, wherein an inner chamfer is formed on a hole wall of an end of the through hole adjacent to the limiting cavity.

20. A blood pump, comprising an impeller and a driving device, the driving device comprising:
a housing assembly provided with a mounting hole; and
a rotating shaft comprising a first shaft section that is fixedly connected to the impeller and rotatably extends through the mounting hole, wherein the first shaft section is provided with an arc-shaped convex surface on a circumference thereof, the arc-shaped convex surface is partially located in the mounting hole, an inflection point of the arc-shaped convex surface is opposite to a hole wall of the mounting hole, and a gap between the arc-shaped convex surface and the hole wall of the mounting hole is minimum at the inflection point of the arc-shaped convex surface, when the first shaft section is in contact with the hole wall of the mounting hole, the inflection point of the arc-shaped convex surface is in contact with the hole wall of the mounting hole; wherein the impeller is fixedly connected to the first shaft section, and the impeller is capable of rotating along with the rotating shaft.
